# EUROPÄISCHE PATENTSCHRIFT

(11) **EP 2 195 075 B1**
(45) Veröffentlichungstag und Bekanntmachung des Hinweises auf die Patenterteilung: **05.07.2017**
(21) Anmeldenummer: 08839672.6
(22) Anmeldetag: 07.10.2008
(51) Int. Cl.: A61M 39/22

(54) **VORRICHTUNG ZUM ZUFÜHREN VON MEDIZINISCHEN FLÜSSIGKEITEN**
DEVICE FOR ADMINISTERING MEDICAL LIQUIDS
DISPOSITIF D'AMENÉE DE LIQUIDES MÉDICAUX

(30) Priorität: 12.10.2007 DE 102007049126
(43) Veröffentlichungstag der Anmeldung: 16.06.2010
(73) Patentinhaber: Fresenius Kabi Deutschland GmbH, 61352 Bad Homburg v.d.H. (DE)
(72) Erfinder: HORNIG, Wolfgang, 58566 Kierspe-Bollwerk (DE); SCHÄFER, Helmut, 61250 Usingen (DE); BREUER-THAL, Barbara, 65510 Idstein (DE); OSTER, Marcel, 41065 Mönchengladbach (DE)
(74) Vertreter: Kusche, Robert
(86) Internationale Anmeldenummer: PCT/EP2008/008429
(87) Internationale Veröffentlichungsnummer: WO 2009/049785

(56) Entgegenhaltungen:
- EP-A- 1 627 658
- EP-A- 1 829 579
- WO-A-00/24313
- WO-A-92/21403
- US-A- 3 603 347

## Beschreibung

Die Erfindung betrifft eine Vorrichtung zum Zuführen von medizinischen Flüssigkeiten, insbesondere zum Applizieren eines Medikaments in die Schlauchleitung eines Überleitgeräts für die enterale oder parenterale Ernährung.

Zur enteralen oder parenteralen Ernährung finden Überleitgeräte Verwendung, mit denen eine Nährlösung aus einem Behälter einem Patienten zugeführt wird. Die bekannten Überleitgeräte weisen eine Schlauchleitung mit Konnektoren auf, die einerseits mit dem Nährlösungsbehälter und andererseits mit einer Ernährungssonde verbunden wird.

Bei der enteralen oder parenteralen Ernährung ist es in einzelnen Fällen erforderlich, in die Schlauchleitung des Überleitgeräts ein Medikament zu applizieren. Wenn das Medikament in die Schlauchleitung des Überleitgeräts während der enteralen oder parenteralen Ernährung appliziert wird, besteht grundsätzlich die Gefahr, dass die applizierte Flüssigkeit nicht in Richtung Patient, sondern in den Nährlösungsbehälter strömt. Dadurch kann die Wirkung des Medikaments verzögert oder durch die Verdünnung mit der Nährlösung der gewünschte Effekt nicht erzielt werden.

Die DE 35 03 044 C2 beschreibt eine Einrichtung zum Verabreichen von Infusionen, Transfusionen oder Injektionen mit einem Mehrwegehahn, der ein Gehäuse mit einem Einlassstutzen zum Zuführten von Infusionen bzw. Transfusionen, einem Auslassstutzen zum Anschluss einer zum Patienten führenden Leitung sowie einem Anschlussstück zum Zuführen eines Medikaments aufweist, wobei die Stutzen um jeweils 90° versetzt zueinander angeordnet sind. In dem Hahngehäuse ist zur Herstellung einer Strömungsverbindung zwischen den einzelnen Stutzen ein Küken mit drei um jeweils 90° versetzten Kanälen gelagert. Bei dem bekannten Mehrwegehahn liegen sämtliche Anschlussstücke in einer Ebene.

Aus der DE 196 50 664 C1 ist eine Vorrichtung zum Applizieren eines Medikaments in die Schlauchleitung eines Überleitgeräts bekannt, die einen Verschlusskörper mit zwei Durchgängen aufweist, der drehbar in einem Basiskörper mit einem Einlass für die Nährlösung und einem Auslass für die Nährlösung gelagert ist. Durch Drehen des Verschlusskörpers kann der Strömungsweg zwischen dem Einlass und Auslass entweder freigegeben oder geschlossen werden. In der geschlossenen Position ist ein zweiter Strömungsweg freigegeben, so dass das Medikament appliziert werden kann. Da in dieser Stellung der Strömungsweg zwischen dem Einlass und Auslass für die Nährlösung unterbrochen ist, kann die applizierte Flüssigkeit nur in eine Richtung, d.h. zum Patienten strömen.

Die Anschlussstücke der bekannten Vorrichtung sind derart angeordnet, dass Einlass und Auslass für die Nährlösung auf einer Achse liegen, während der Einlass für das Medikament auf einer dazu senkrecht stehenden Achse liegt. Folglich wird das Medikament im rechten Winkel zu der strömenden Nährlösung zugespritzt.

Auch die EP-A-1 627 658 beschreibt eine Vorrichtung zum Zuführen von medizinischen Flüssigkeiten mit einem Verschlusskörper, der in einem Basiskörper drehbar angeordnet ist. Der Verschlusskörper weist zwei Durchgänge auf, von denen der eine Durchgang in einer ersten Position den Einlass des Basiskörpers mit dem Auslass des Basiskörpers verbindet und der andere Durchgang in einer zweiten Position den Einlass des Verschlusskörpers mit dem Auslass des Basiskörpers verbindet.

Die bekannten Vorrichtungen zum Zuführen von medizinischen Flüssigkeiten, die über einen Basiskörper und einen Verschlusskörper verfügen, der in dem Basiskörper drehbar ist, zeichnen sich dadurch aus, dass die beiden Durchgänge, die eine Flüssigkeitsverbindung zwischen dem Einlass des Basiskörpers oder dem Einlass des Verschlusskörpers einerseits und dem Auslass des Basiskörpers andererseits schaffen, auf einer Ebene angeordnet sind, so dass eine Drehung des Verschlusskörpers ausreicht, um den Einlass des Basis- oder Verschlusskörpers mit dem Auslass des Basiskörpers zu verbinden. Dies setzt aber der Ausbildung der Durchgänge Grenzen, so dass die Strömungsführung in dem Basis- und Verschlusskörper nicht optimal ist. Es können sich Bereiche geringer Flüssigkeitsströmung ergeben oder Zonen entstehen, in denen Verwirbelungen auftreten.

Aus der US-A-3 603 347 ist eine Vorrichtung zum Zuführen von medizinischen Flüssigkeiten gemäß dem Oberbegriff des Anspruchs 1 bekannt, die auch über einen Basiskörper und einen Verschlusskörper verfügt, wobei der Verschlusskörper in dem Basiskörper drehbar angeordnet ist. Auch kann der Verschlusskörper in dem Basiskörper eine Hubbewegung ausführen. Durch Drehen des Verschlusskörpers einerseits bzw. Absenken des Verschlusskörpers andererseits wird ein Einlass mit einem ersten Auslass verbunden oder der Einlass mit einem zweiten Auslass verbunden. Die Drehbewegung des Verschlusskörpers ist aber nicht mit einer gleichzeitigen Hubbewegung verbunden. Daher ist es erforderlich, sowohl durch Verschwenken des Griffteils den Verschlusskörper anzuheben bzw. abzusenken als auch durch Drehen des Griffteils den Verschlusskörper zu drehen.
Die WO 00/24313 A beschreibt eine Ventilanordnung, bei der zwar der Verschlusskörper auch angehoben bzw. abgesenkt wird, die Drehbewegung des Verschlusskörpers ist aber wieder nicht mit einer gleichzeitigen Hubbewegung verbunden.
Der Erfindung liegt die Aufgabe zugrunde, eine einfach zu handhabende Vorrichtung mit einer verbesserten Strömungsführung zu schaffen, die es erlaubt, medizinische Flüssigkeiten zuzuführen, insbesondere in eine Schlauchleitung eines Überleitgeräts zur enteralen und parenteralen Ernährung zu applizieren, ohne dass die Gefahr besteht, dass die Flüssigkeit in die falsche Richtung fließt.
Die Lösung dieser Aufgabe erfolgt erfindungsgemäß mit den Merkmalen des Patentanspruchs 1.

Die erfindungsgemäße Vorrichtung zum Zuführen von Flüssigkeiten, insbesondere zum Applizieren eines Medikaments in die Schlauchleitung eines Überleitgeräts weist einen Basiskörper und einen Verschlusskörper auf, der in einer zylindrischen Ausnehmung des Basiskörpers drehbar ist. Der Basiskörper weist einen Einlasskanal und Auslasskanal auf, während der Verschlusskörper einen Einlasskanal aufweist. In Abhängigkeit von der Position, in die der Verschlusskörper gedreht wird, sind entweder Einlasskanal und Auslasskanal des Basiskörpers oder Einlasskanal des Verschlusskörpers und Auslasskanal des Basiskörpers miteinander verbunden. Von Vorteil ist, dass der Strömungsweg für die Nährlösung und der Strömungsweg für das Medikament bei der erfindungsgemäßen Vorrichtung getrennt sind. So kann weder das Medikament in den Durchgang für die Nährlösung noch Nährlösung in den Durchgang für das Medikament fließen.
Die erfindungsgemäße Vorrichtung zeichnet sich dadurch aus, dass der Verschlusskörper beim Drehen gleichzeitig in Folge dieses Drehens eine axiale Hubbewegung ausführt, wobei der erste und zweite Durchgang derart in dem Verschlusskörper angeordnet sind, dass in der ersten Position der erste Durchgang des Verschlusskörpers den Einlasskanal mit dem Auslasskanal des Basiskörpers verbindet und in der zweiten Position des Verschlusskörpers den Einlasskanal des Verschlusskörpers mit dem Einlasskanal des Basiskörpers verbindet. Die Hubbewegung des Verschlusskörpers schafft einen relativ großen Freiraum, was die Ausbildung der beiden Durchgänge in dem Verschlusskörper betrifft, da der Einlass- und Auslass der beiden Durchgänge des Verschlusskörpers mit dem Einlass bzw. Auslass der zugehörigen Kanäle des Basiskörpers nicht auf einer Ebene zu liegen brauchen, sondern auf unterschiedlichen Höhen angeordnet werden können. Die Hubbewegung des Verschlusskörpers erweist sich insofern als vorteilhaft, als unterschiedliche Höhen von Ein- und Auslass der Durchgänge bzw. der Kanäle ausgeglichen werden können. So können die Durchgänge derart ausgebildet werden, dass sich eine optimale Strömungsführung ergibt, bei der keine Verwirbelungen auftreten und keine Zonen entstehen, in denen die Flüssigkeitsströmung gering ist.

Bei der erfindungsgemäßen Vorrichtung kann der Durchgang für das Medikament oberhalb des Durchgangs für die Nährlösung angeordnet sein. Da sich beide Durchgänge nicht kreuzen, kann der Durchgang für die Nährlösung derart ausgebildet werden, dass der Durchgang einen geradlinigen Verlauf hat. Daraus kann sich auch eine höhere Applikationsgeschwindigkeit ergeben. Beide Durchgänge in dem Verschlusskörper können als durchgehende Bohrungen oder Ausnehmungen in dem Verschlusskörper ausgebildet sein. Allein entscheidend ist, dass die Durchgänge eine Strömungsverbindung herstellen.

Darüber hinaus erweist sich als vorteilhaft, dass die Anwender im Bereich der enteralen Ernährung beim Drehen eines Verschlusskörpers bereits an eine Hubbewegung gewohnt sind, da auch beim Verschließen der so genannten Luer-Lock-Konnektoren, die in der Medizin eine breite Anwendung finden, die Verschlussorgane eine Hubbewegung ausüben. Im Übrigen kann der Anwender schon an der Hubbewegung intuitiv erkennen, ob die Vorrichtung beim Drehen des Verschlusskörpers geöffnet oder verschlossen wird.

Der Basiskörper der erfindungsgemäßen Vorrichtung ist mit den beiden Kanälen für den Einlass und Auslass der Flüssigkeit vorteilhafterweise spiegelsymmetrisch aufgebaut. Mit der Montage des Verschlusskörpers wird dann die Funktion der Kanäle als Einlass- bzw. Auslasskanal festgelegt. Da die beiden Teile vorteilhafterweise in ihren Passungen symmetrisch zueinander aufgebaut sind, ist es unerheblich, in welcher Position die beiden Teile zueinander montiert werden, was die Montage vereinfacht.

Bei einer bevorzugten Ausführungsform der erfindungsgemäßen Vorrichtung ist der erste Durchgang in dem Verschlusskörper, der den Einlasskanal des Basiskörpers, insbesondere zum Zuführen der Nährlösung, mit dem Auslasskanal des Basiskörpers verbindet, eine Bohrung in dem Verschlusskörper, deren Längsachse quer zur Drehachse des Verschlusskörpers verläuft. Während der enteralen oder parenteralen Ernährung ist also eine geradlinige Strömung der Nährlösung durch die Vorrichtung gegeben. Damit bleiben die Strömungsverhältnisse in der Schlauchleitung des Überleitgeräts während der enteralen oder parenteralen Ernährung durch den Einsatz der erfindungsgemäßen Vorrichtung weitgehend unverändert. Anstelle einer Bohrung kann der Verschlusskörper zur Schaffung eines Durchgangs an seinem unteren Ende aber auch U-förmig ausgebildet sein, wobei die gegenüberliegenden Schenkel des U-förmigen Endes eine Ausnehmung seitlich umschließen, durch die Flüssigkeit strömen kann.

Bei einer weiteren bevorzugten Ausführungsform weist der zweite Durchgang, der den Einlass für das Medikament mit dem Auslass der Vorrichtung verbindet, einen in Richtung der Drehachse verlaufenden Kanal aufweist, an den sich ein quer zu der Drehachse verlaufender Kanal anschließt. Der Übergangsbereich zwischen diesen beiden Abschnitten kann unterschiedlich ausgebildet sein, sollte aber keine Engstellen und Toträume aufweisen. Zwar ist dieser Strömungsweg für die medizinische Flüssigkeit nicht geradlinig. Dies ist aber beim Zuspritzen eines Medikaments unerheblich. Allein entscheidend ist, dass die Nährlösung ungehindert durch die erfindungsgemäße Vorrichtung strömen kann.

Eine weitere bevorzugte Ausfuhrungsform sieht vor, dass der Auslass des ersten Durchgangs mit dem Auslass des zweiten Durchgangs einen rechten Winkel einschließt. Daher reicht eine Drehung um 90° aus, um die jeweilige Strömungsverbindung herzustellen. Es ist aber auch möglich, dass der Auslass des ersten und zweiten Durchgangs einen größeren oder kleineren Winkel einschließen, wodurch sich ein größerer oder kleinerer Winkel ergibt, um den der Verschlusskörper zum Öffnen oder Schließen gedreht werden muss.

Eine weitere besonders bevorzugte Ausführungsform sieht vor, dass der Basiskörper ein im Wesentlichen zylindrisches Teilstück aufweist, an das sich radial nach außen vorstehende Anschlussstücke anschließen, in denen der Einlasskanal und der Auslasskanal des Basiskörpers ausgebildet sind. Der Verschlusskörper weist ein Anschlussstück auf, das sich vorzugsweise quer zu den Anschlussstücken des Basiskörpers erstreckt, wobei der Einlasskanal des Verschlusskörpers in dem Anschlussstück ausgebildet ist. Folglich liegen der Einlass für die Nährlösung und der Auslass der Vorrichtung auf einer Achse, während die medizinische Flüssigkeit im rechten Winkel zu der durch die Vorrichtung strömenden Nährlösung zugespritzt werden kann.

Das Anschlussstück des Verschlusskörpers weist vorzugsweise einen Innenkonus auf, um beispielsweise eine Blasenspritze oder einen Stufenadapter anschließen zu können. Vorzugsweise ist ein Innenkonus mit einem größeren oder kleineren Durchmesser vorgesehen, der entweder den Anschluss einer Blasenspritze oder eines Stufenadapters erlaubt. Grundsätzlich ist es aber auch möglich, einen Luer-Konus oder Luer-Lock-Gewinde an dem Anschlussstück vorzusehen.

Der Verschlusskörper wird vorzugsweise an dem Basiskörper dadurch unverlierbar gesichert, dass der Verschlusskörper einen äußeren Abschnitt aufweist, der den oberen Abschnitt des Basiskörpers übergreift.

Die Hubbewegung wird beim Drehen des Verschlusskörpers vorzugsweise mit einer Kulissenführung erreicht. Der den oberen Abschnitt des Basiskörpers übergreifende Abschnitt des Verschlusskörpers weist bei einer bevorzugten Ausführungsform mindestens eine, vorzugsweise zwei umfangsmäßig verteilt angeordnete Nuten auf, in die jeweils ein Absatz des Basiskörpers greift. Der Verlauf der jeweiligen Nut ist derart beschaffen, dass der Verschlusskörper beim Drehen die Hubbewegung entweder nach oben oder nach unten ausführt, d.h. sich nach oben oder unten verschiebt. Dabei kann die Nut über ihren Verlauf entlang des Umfangs unterschiedliche Steigungen haben, was das Öffnungs- oder Schließverhalten beeinflusst. Vorzugsweise erstreckt sich die Nut über einen Umfangswinkel von 90°, so dass der Verschlusskörper in dem Basiskörper um 90° gedreht werden kann.

Um den Verschlusskörper besser greifen zu können, weist der Verschlusskörper zwei Griffelemente auf, die radial von dem Verschlusskörper abstehen. Vorzugsweise sind die Griffelemente als flache Körper ausgebildet, die sich bis an die Anschlussstücke des Basiskörpers erstrecken, wobei der verbleibende Spalt zwischen Griffelelement und Anschlussstück so klein wie möglich sein sollte. Dadurch wird sichergestellt, dass sich die Schlauchleitung nicht in dem verbleibenden Spalt verklemmt.

Die Griffelemente weisen an der den Anschlussstücken zugewandten Seite vorzugsweise eine schräg zu den Anschlusstücken weg verlaufende Kontur auf. Diese Abschrägung verbessert die Handhabung, da die Griffflächen in diesem Bereich nicht die den Basiskörper haltenden Finger berühren können.

Zur Vermeidung von Verwechselungen zwischen den Anschlussstücken des Basiskörpers kann mindestens eines der Anschlussstücke mit einer erhabenen Struktur, insbesondere mit einem erhabenen Streifen versehen sein, der sich leicht ertasten lässt. Die Griffelemente können zur Signalisierung der Flussrichtung mit muldenförmigen Vertiefungen oder Aussparungen gekennzeichnet sein. Es ist aber auch möglich, die Griffelemente mit einer erhabenen Struktur zu kennzeichnen.

Im Folgenden wird ein Ausführungsbeispiel der Erfindung unter Bezugnahme auf die Zeichnungen im Einzelnen erläutert.

Es zeigen:
- Fig. 1: ein Ausführungsbeispiel der erfindungsgemäßen Vorrichtung zum Zuführen von Flüssigkeiten in perspektivischer Darstellung, wobei der Einlass für die Nährlösung mit dem Auslass der Vorrichtung verbunden ist,
- Fig. 2: einen Schnitt durch die Vorrichtung von Fig. 1,
- Fig. 3: die Vorrichtung von Fig. 1 in perspektivischer Darstellung, wobei der Einlass für das Medikament mit dem Auslass der Vorrichtung verbunden ist,
- Fig. 4: einen Schnitt durch die Vorrichtung von Fig. 3,
- Fig. 5: die Vorrichtung von Fig. 1 mit einem Stufenadapter in geschnittener Darstellung,
- Fig. 6: die Vorrichtung von Fig. 1 in geschnittener Darstellung mit einer Blasenspritze,
- Fig. 7: der Verschlusskörper eines Ausführungsbeispiels der erfindungsgemäßen Vorrichtung mit einem Verschlussstopfen,
- Fig. 8: ein weiteres Ausführungsbeispiel der erfindungsgemäßen Vorrichtung in geschnittener Darstellung, wobei der Einlass für die Nährlösung mit dem Auslass der Vorrichtung verbunden ist und
- Fig. 9: die Vorrichtung von Fig. 8 in geschnittener Darstellung, wobei der Einlass für das Medikament mit dem Auslass der Vorrichtung verbunden ist.

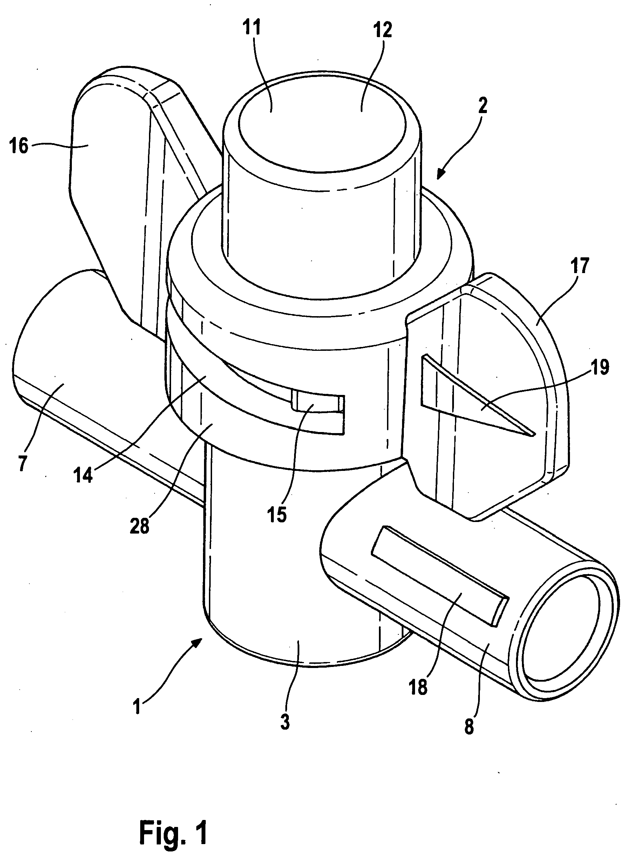

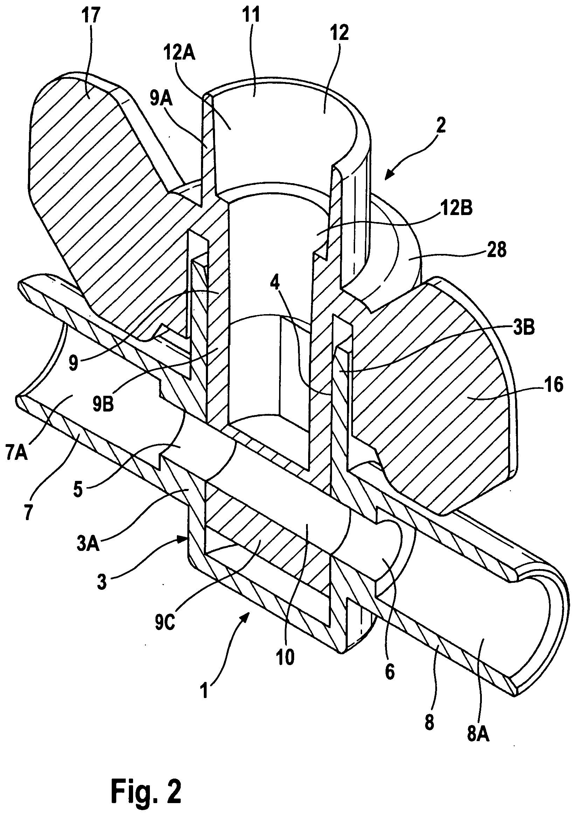

Fig. 1 zeigt in perspektivischer Darstellung ein Ausführungsbeispiel der erfindungsgemäßen Vorrichtung zum Applizieren eines Medikaments in die nicht dargestellte Schlauchleitung eines nicht dargestellten Überleitgeräts zur enteralen oder parenteralen Ernährung. Die Applikationsvorrichtung verfügt über einen Basiskörper 1 und einen Verschlusskörper 2. Basis- und Verschlusskörper 1, 2 sind Spritzgießteile, die kostengünstig in großen Stückzahlen hergestellt werden können. Fig. 2 zeigt die Vorrichtung von Fig. 1 in geschnittener Darstellung.

Der Basiskörper 1 weist ein im Wesentlichen zylindrisches Teilstück 3 mit einer im Wesentlichen zylindrischen Ausnehmung 4 auf. In der Zylinderwand des unteren Teilstücks 3A des Basiskörpers 3 sind zwei Bohrungen 5, 6 vorgesehen, die sich einander axial gegenüberliegen.

An die Bohrung 5 schließt sich ein hohlzylindrisches Teilstück 7 und an die Bohrung 6 schließt sich ein hohlzylindrisches Teilstück 8 an, die zu beiden Seiten radial von dem Basiskörper 3 nach außen vorstehen. Das hohlzylindrische Teilstück 7 bildet ein Anschlussstück 7 mit einem Einlasskanal 7A, an das der Abschnitt der Schlauchleitung des Überleitgeräts angeschlossen wird, der von dem Behälter zur Aufnahme der Nährlösung zu der erfindungsgemäßen Vorrichtung führt. Das hohlzylindrische Teilstück 8 bildet ein Anschlussstück 8 mit einem Auslasskanal 8A, an das der Schlauchleitungsabschnitt angeschlossen wird, der von der erfindungsgemäßen Applikationsvorrichtung zu der Ernährungssonde führt. Die beiden nicht dargestellten Schlauchleitungsabschnitte können in die Anschlussstücke 7, 8 eingeschoben und mit den Anschlussstücken verschweißt und/oder verklebt werden.

Der Verschlusskörper 2 weist ein im Wesentlichen zylindrisches Teilstück 9 mit einem oberen Abschnitt 9A und einem unteren Abschnitt 9C auf, zwischen denen ein mittlerer Abschnitt 9B liegt. Das zylindrische Teilstück 9 des Verschlusskörpers 2 ist in das zylindrische Teilstück 3 des Basiskörpers 1 eingesetzt ist, wobei der Verschlusskörper in dem Basiskörper um die Längsachse des zylindrischen Teilstücks drehbar ist.

Durch den unteren Abschnitt 9C des zylindrischen Teilstücks 9 des Verschlusskörpers 2 erstreckt sich eine quer zu der Längsachse verlaufende Bohrung 10. Der Auslass der Bohrung 10 fluchtet in der in den Fig. 1 und 2 gezeigten Position des Verschlusskörpers 2 mit dem Einlasskanal 7A des Basiskörpers 1 und der Auslass der Bohrung 10 des Verschlusskörpers mit dem Auslasskanal 8A des Basiskörpers 1. Dadurch wird ein Durchgang 10 geschaffen, der das einlassseitige Anschlussstück 7 mit dem auslassseitigen Anschlussstück 8 verbindet. Somit kann Nährlösung ungehindert durch die Applikationsvorrichtung strömen, wobei die Strömung einen geradlinigen Verlauf hat.

Der obere Abschnitt 9A des zylindrischen Teilstücks 9 des Verschlusskörpers 2 bildet ein Anschlussstück 11 für eine Schlauchleitung, in dem ein Einlasskanal 12 für das Medikament ausgebildet ist, der sich in axialer Richtung durch den oberen Abschnitt 9A des zylindrischen Teilstücks 9 bis zu dem mittleren Abschnitt 9B erstreckt.

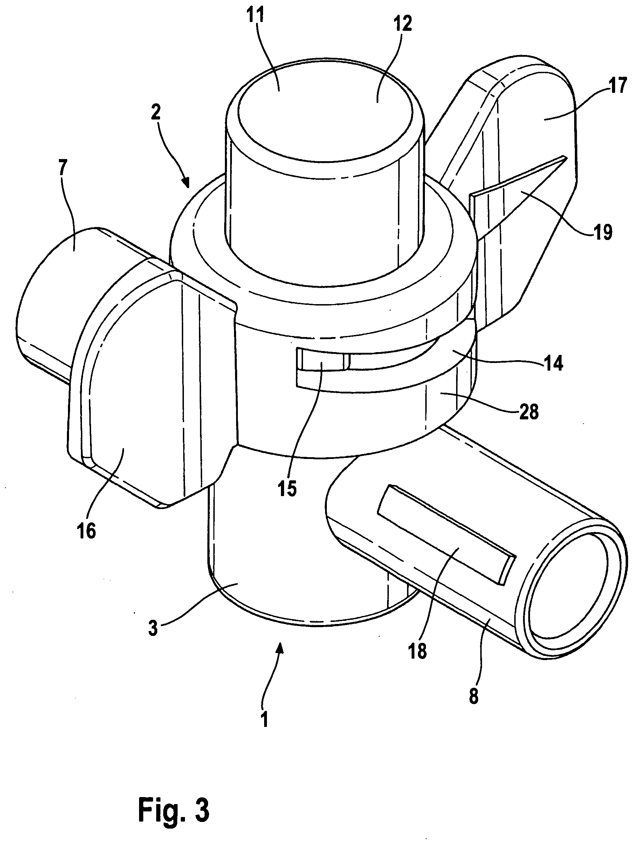

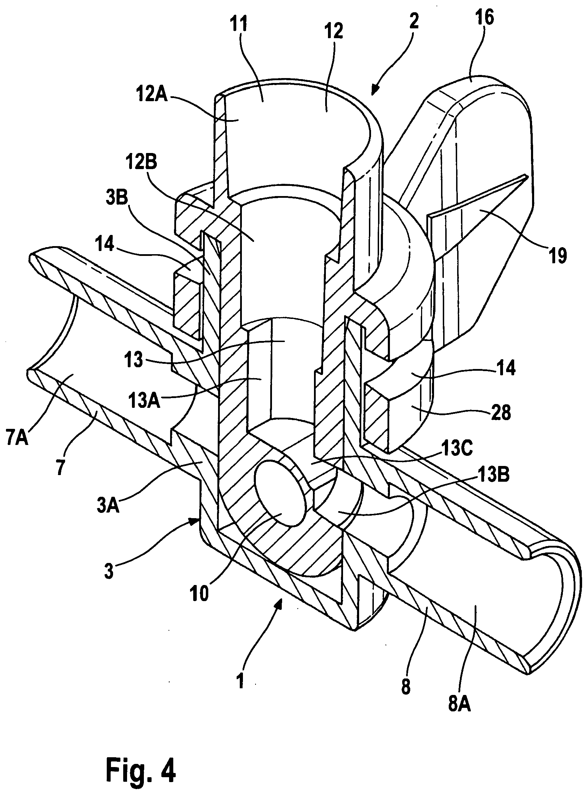

Die Fig. 3 und 4 zeigen den Verschlusskörper in der zweiten Position, wobei der Verschlusskörper gegenüber der in den Figuren 1 und 2 gezeigten Position entgegen dem Uhrzeigersinn um 90° verdreht ist. In dieser Position fluchten der Einlasskanal 7A und der Auslasskanal 8A des Basiskörpers 1 nicht mit der Bohrung 10 des Verschlusskörpers 2, so dass die Strömungsverbindung zwischen dem einlassseitigen Anschlussstück 7 und dem auslassseitigen Anschlussstück 8 unterbrochen ist.

Neben dem ersten Durchgang 10, der den Einlasskanal 7A mit dem Auslasskanal 8A des Basiskörpers 1 verbindet, weist der Verschlusskörper einen zweiten Durchgang 13 auf, der in der in den Figuren 3 und 4 gezeigten Position den Einlasskanal 12 des Verschlusskörpers 2 mit dem Auslasskanal 7A des Basiskörpers 1 verbindet.

Der zweite Durchgang 13 weist einen sich an den Einlasskanal 12 des Verschlusskörpers 2 anschließenden, axial verlaufenden Abschnitt 13A auf, der in einen sich quer zu der Drehachse verlaufenden Abschnitt 13B übergeht, wobei der Auslass des quer zu der Drehachse verlaufenden Abschnitts 13B mit der quer verlaufenden Bohrung 10 in dem Verschlusskörper 2 einen Umfangswinkel von 90° einschließt. Die beiden Abschnitte 13A, 13B des zweiten Durchgangs 13 in dem Verschlusskörper verbindet ein bogenförmiger Übergangskanal 13C, der sich um die axiale Bohrung 10 in dem unteren Abschnitt des Verschlusskörpers erstreckt. Der obere Teil des axial verlaufenden Abschnitts 13A ist im Wesentlichen zylindrisch, während der Abschnitt 13A in dem unteren Teil zur leichteren Entformung beim Spritzgießen zwei parallele Flächen hat.

In der in den Figuren 1 und 2 gezeigten Position weist der Auslass des zweiten Durchgangs 13 auf die Wandung der zylindrischen Ausnehmung 12 des Basiskörpers 1. Daher ist der zweite Durchgang verschlossen, so dass keine Flüssigkeit von dem Einlasskanal 12 des Verschlusskörpers 2 zu dem Auslasskanal 8A des Basiskörpers 1 strömen kann, während Flüssigkeit durch den ersten Durchgang 10 des Verschlusskörpers strömt.

In der in den Figuren 3 und 4 gezeigten Position hingegen weist der Auslass des zweiten Durchgangs 13 auf den Auslasskanal 8A des Basiskörpers 1, wodurch eine Flüssigkeitsverbindung zwischen dem Einlasskanal 12 des Verschlusskörpers und dem Auslasskanal 8A des Basiskörpers 1 hergestellt wird.

Der erste und der zweite Durchgang 10, 13 sind in dem Verschlussköper 2 derart ausgebildet, dass der Auslass des ersten Durchgangs 10 und der Auslass des zweiten Durchgangs 13 auf unterschiedlichen Höhen liegen. Dabei ist der Auslass des zweiten Durchgangs oberhalb des Ausgangs des ersten Durchgangs angeordnet. Zum Ausgleich dieses Höhenversatzes führt der Verschlusskörper beim Drehen zwischen den beiden Positionen, d.h. über einen Umfangswinkel von 90°, eine Hubbewegung aus. Dabei entspricht die Höhe der Hubbewegung dem axialen Abstand zwischen dem Auslass des ersten Durchgangs und dem Auslass des zweiten Durchgangs. In der gezeigten Position wird der Verschlusskörper zum Verschließen der Strömungsverbindung zwischen Einlass-und Auslasskanal des Basiskörpers also nach unten (Figuren 3 und 4) und zum Öffnen der Strömungsverbindung nach oben (Figuren 1 und 2) verschoben.

Der Verschlusskörper 2 ist in dem Basiskörper 1 unverlierbar gesichert, wobei der Verschlusskörper nur um einen Winkel von 90° gedreht werden kann. Der Verschlusskörper 2 weist einen äußeren Abschnitt 28 auf, der sich an den oberen Abschnitt 9A des Teilstücks 9 des Verschlusskörpers anschließt und den oberen Abschnitt 3B des Basiskörpers 2 übergreift.

An dem äußeren Abschnitt 28 des Verschlusskörpers 2, der den Basiskörper 1 übergreift, sind zwei umlaufende Nuten 14 vorgesehen, die sich jeweils über einen Umfangswinkel von 90° erstrecken. In die umlaufenden Nuten 14 greifen Absätze 15, die von dem oberen Teilstück 3B des Basiskörpers 1 abstehen. Die Nuten 14 und Absätze 15 bilden eine Kulissenführung, die derart ausgebildet ist, dass der Verschlusskörper 2 in dem Basiskörper 1 nur zwischen den beiden in den Fig. 1 und 2 bzw. 3 und 4 gezeigten Positionen verdreht werden kann und während der Drehbewegung die erforderliche Hubbewegung ausführt. In diesen beiden Positionen schlagen die Absätze des Basiskörpers an dem jeweiligen Ende der Nuten an.

An dem äußeren Abschnitt 28 des Verschlusskörpers 2 sind zwei radial abstehende Griffelemente 16, 17 angeformt, die in der in den Fig. 1 und 2 gezeigten Positionen des Verschlusskörpers 2 in die Richtung der Anschlussstücke 7, 8 zeigen und in der in den Fig. 3 und 4 gezeigten Position im rechten Winkel dazu angeordnet sind. Beide Griffelemente 16, 17 sind flache Körper, die sich bis nahe an den oberen Rand der Anschlussstücke und des Basiskörpers erstrecken. Da ein größerer Spalt zwischen dem unteren Rand der Griffelemente und dem oberen Rand der Anschlussstücke nicht gegeben ist, besteht nicht die Gefahr, dass sich die Schlauchleitung des Überleitgeräts darin verklemmt. Die Griffelemente 16, 17 weisen an der den Anschlussstücken zugewandten Seite eine schräg von den Anschlussstücken weg verlaufende Kontur auf, so dass sie in diesem Bereich die den Basiskörper haltenden Finger nicht berühren können.

Das auslassseitige Anschlussstück 8 ist an der Außenseite mit einem erhabenen Streifen 18 versehen, der mit den Fingern ertastet werden kann, so dass der Einlass vom Auslass leicht unterschieden werden kann. Weiterhin ist eines der Griffelemente 17 zur Kennzeichnung der Flussrichtung mit einem Pfeil 19 gekennzeichnet, der als erhabene Struktur, als muldenförmige Vertiefung oder als Aussparung ausgebildet sein kann.

Bei der Montage des Verschlusskörpers 2 ist grundsätzlich unerheblich, in welcher Position der Verschlusskörper in den Basiskörper 1 eingesetzt wird, da Basiskörper und Verschlusskörper bezüglich der mittleren Schnittebene symmetrisch ausgebildet sind.

Der Einlasskanal 12 in dem Anschlussstück 11 des Verschlusskörpers 2 ist stufenförmig ausgebildet und weist einen oberen konischen Abschnitt 12A mit einem größeren Innendurchmesser und einen unteren konischen Abschnitt 12B mit einem kleineren Innendruchmesser auf. Das Anschlussstück des Verschlusskörpers kann mit einem in den Figuren 1 bis 4 nicht dargestellten Stufenadapter zum Anschluss einer weiteren Schlauchleitung zum Zuführen des Medikaments oder mit dem Anschlusstrichter einer Blasenspritze verbunden werden.

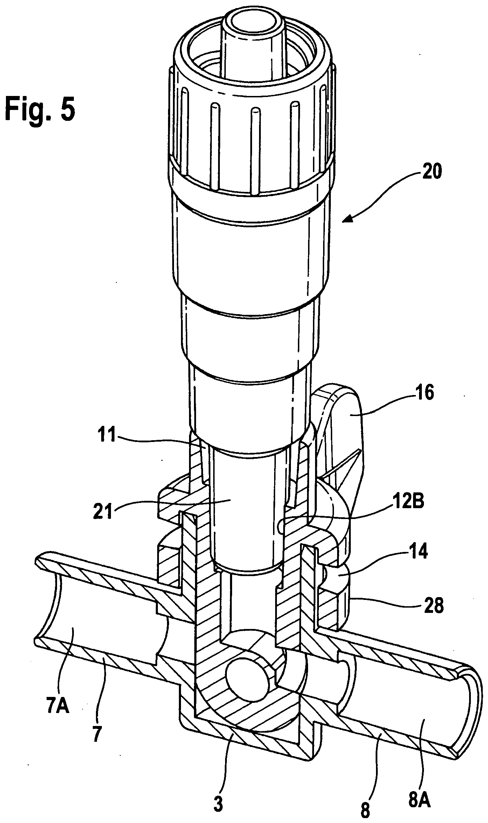

Fig. 5 zeigt ein Ausführungsbeispiel, bei dem ein Stufenadapter 20 an dem Anschlussstück 11 der erfindungsgemäßen Vorrichtung angeschlossen ist. Der Stufenadapter verfügt über einen kegelförmigen Anschlusskonus 21, dessen Konizität der Konizität des unteren Innenkonus 12B des Anschlussstücks entspricht.

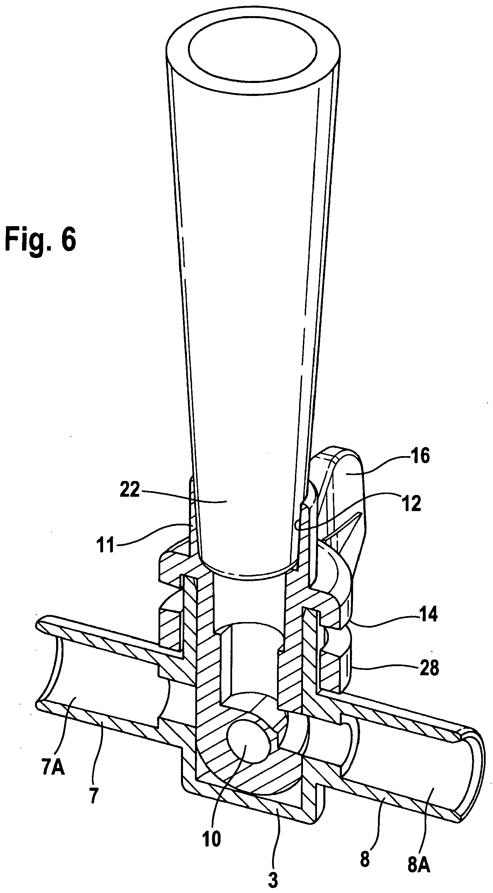

Fig. 6 zeigt ein Ausführungsbeispiel, bei dem der Anschlusskonus 22 einer nicht dargestellten Blasenspritze an dem Anschlussstück 11 der erfindungsgemäßen Vorrichtung angeschlossen ist. Hier entspricht die Konizität des Anschlusskonus der Blasenspritze der Konizität des oberen Innenkonus 12A des Anschlussstücks der erfindungsgemäßen Vorrichtung.

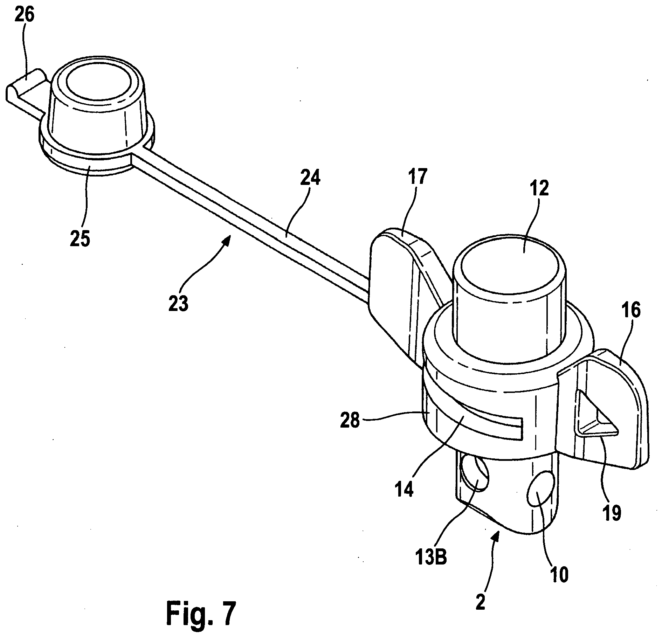

Fig. 7 zeigt den Verschlusskörper einer Ausführungsform der erfindungsgemäßen Vorrichtung in perspektivischer Darstellung, der sich von dem unter Bezugnahme auf die Figuren 1 bis 6 beschriebenen Ausführungsbeispiel dadurch unterscheidet, der Verschlusskörper 2 mit einem Verschlussteil 23 verschlossen werden kann. Die einander entsprechenden Teile sind in den Figuren mit den gleichen Bezugszeichen versehen. Der Verschlussteil weist eine flexible Lasche 24 auf, die an den äußeren Abschnitt 28 des Verschlusskörpers angeformt ist. An die flexible Lasche 24 ist ein Verschlussstopfen 25 angeformt, der eine Grifflasche 26 aufweist. Zum Verschließen des Verschlusskörpers wird der Verschlussstopfen in den Einlasskanals 12 des Verschlusskörpers gedrückt.

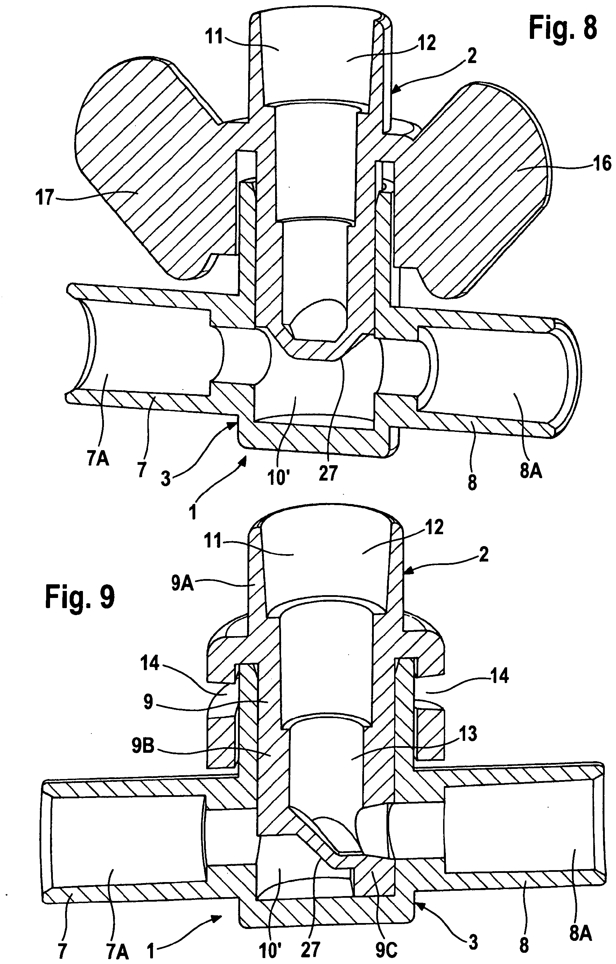

Die Figuren 8 und 9 zeigen eine weitere Ausführungsform der erfindungsgemäßen Vorrichtung, die sich von den oben beschriebenen Ausführungsbeispielen nur durch die Ausbildung des unteren Abschnitts 9C des Verschlusskörpers 2 unterscheidet. Die einander entsprechenden Teile sind wieder mit den gleichen Bezugszeichen versehen.

Bei der Ausführungsform von den Figuren 8 und 9 weist der untere Abschnitt 9C des Verschlusskörpers 9 nicht eine axiale Bohrung auf, sondern der erste Durchgang durch den Verschlusskörper wird durch einen Rücksprung 27 des unteren Abschnitts 9C des Verschlusskörpers 9 geschaffen, der an der dem Auslass des zweiten Durchgangs 13 gegenüberliegenden Seite in einer Ebene unterhalb des Auslasses liegt. Fig. 8 zeigt die Position des Verschlusskörpers, in der der erste Durchgang 10', der durch den Rücksprung 27 gebildet wird, mit dem Auslasskanal 8A des Basiskörpers fluchtet. Durch Drehen des Verschlusskörpers entgegen des Uhrzeigersinns um 90° wird der Auslass des zweiten Durchgangs 13 mit dem Auslasskanal 8A des Basiskörpers 1 verbunden. Bei der Drehbewegung wird der Verschlusskörper 2 um den Höhenversatz der Auslässe des ersten und zweiten Durchgangs 10', 13 abgesenkt, so das der Auslass des zweiten Durchgangs 13 und der Auslasskanal 8A in der in Fig. 9 gezeigten Position auf einer Höhe liegen.

## Patentansprüche

1. Vorrichtung zum Zuführen von medizinischen Flüssigkeiten, insbesondere zum Applizieren eines Medikaments in die Schlauchleitung eines Überleitgeräts für die enterale oder parenterale Ernährung, mit
einem Basiskörper (1), der einen Einlasskanal (7A) und einen Auslasskanal (8A) aufweist und einem in dem Basiskörper drehbar angeordneten Verschlusskörper (2), der einen Einlasskanal (11) aufweist,
wobei der Verschlusskörper einen ersten Durchgang (10, 10') aufweist, der in einer ersten Position den Einlasskanal des Basiskörpers mit dem Auslasskanal des Basiskörpers verbindet, und einen zweiten Durchgang (13) aufweist, der in einer zweiten Position den Einlasskanal des Verschlusskörpers mit dem Auslasskanal des Basiskörpers verbindet,
**dadurch gekennzeichnet, dass**
der Verschlusskörper (2) derart drehbar in dem Basiskörper (1) angeordnet ist, dass der Verschlusskörper beim Drehen gleichzeitig in Folge dieses Drehens eine axiale Hubbewegung ausführt, wobei
der erste und zweite Durchgang (10, 13) derart in dem Verschlusskörper angeordnet sind, dass in der ersten Position der erste Durchgang (10) den Einlasskanal (7A) des Basiskörpers (1) mit dem Auslasskanal (8B) des Basiskörpers verbindet und in der zweiten Position des Verschlusskörpers den Einlasskanal (11) des Verschlusskörpers (2) mit dem Auslasskanal (8B) des Basiskörpers verbindet.

2. Vorrichtung nach Anspruch 1, **dadurch gekennzeichnet, dass** der erste Durchgang eine Bohrung (10) in dem Verschlusskörper (2) ist, deren Längsachse quer zur Drehachse des Verschlusskörpers verläuft.

3. Vorrichtung nach Anspruch 1 oder 2, **dadurch gekennzeichnet, dass** der zweite Durchgang (13) einen in Richtung der Drehachse des Verschlusskörpers verlaufenden Abschnitt (13A) aufweist, an den sich ein quer zu der Drehachse verlaufender Abschnitt (13B) anschließt.

4. Vorrichtung nach einem der Ansprüche 1 bis 3, **dadurch gekennzeichnet, dass** der Auslass des ersten Durchgangs (10) und der Auslass des zweiten Durchgangs (13) einen Umfangswinkel von 90° einschließen.

5. Vorrichtung nach einem der Ansprüche 1 bis 4, **dadurch gekennzeichnet, dass** der Auslass des ersten Durchgangs (10) und der Auslass des zweiten Durchgangs (13) in axialer Richtung in unterschiedlichen Höhen angeordnet sind, wobei der Abstand zwischen dem Auslass des ersten Durchgangs und dem Auslass des zweiten Durchgangs der Höhe der Hubbewegung entspricht, die der Verschlusskörper beim Drehen zwischen der ersten und zweiten Position ausführt.

6. Vorrichtung nach einem der Ansprüche 1 bis 5, **dadurch gekennzeichnet, dass** der Basiskörper (1) ein im Wesentlichen zylindrisches Teilstück (3) aufweist, an das sich radial nach außen vorstehende Anschlussstücke (7, 8) anschließen, in denen der Einlasskanal und der Auslasskanal des Basiskörpers ausgebildet sind, und dass der Verschlusskörper (2) ein Anschlussstück (12) aufweist, das sich quer zu den Anschlussstücken des Basiskörpers erstreckt, wobei der Einlasskanal (11) des Verschlusskörpers in dem Anschlussstück (12) ausgebildet ist.

7. Vorrichtung nach Anspruch 6, **dadurch gekennzeichnet, dass** das Anschlussstück (12) des Verschlusskörpers (2) einen ersten Abschnitt (12A) aufweist, an den sich ein zweiter Abschnitt (12B) anschließt, wobei der Einlasskanal (11) in dem ersten Abschnitt einen größeren Innendruchmesser als in dem zweiten Abschnitt des Anschlussstücks hat.

8. Vorrichtung nach Anspruch 7, **dadurch gekennzeichnet, dass** mindestens einer der beiden Abschnitte (12A, 12B) des Anschlussstücks (12) konisch ausgebildet ist.

9. Vorrichtung nach einem der Ansprüche 1 bis 8, **dadurch gekennzeichnet, dass** der Verschlusskörper (2) einen äußeren Abschnitt (28) aufweist, der den oberen Abschnitt des Basiskörpers (1) übergreift.

10. Vorrichtung nach Anspruch 9, **dadurch gekennzeichnet, dass** der den oberen Abschnitt des Basiskörpers (1) übergreifende Abschnitt (28) des Verschlusskörpers (2) mindestens eine Nut (14) aufweist, in die ein Absatz (15) des Basiskörpers (1) greift, wobei die Verlauf der Nut derart beschaffen ist, dass der Verschlusskörper beim Drehen eine Hubbewegung ausführt.

11. Vorrichtung nach einem der Ansprüche 1 bis 10, **dadurch gekennzeichnet, dass** der Verschlusskörper (2) zwei Griffelemente (16, 17) aufweist, die radial von dem Verschlusskörper abstehen.

12. Vorrichtung nach Anspruch 11, **dadurch gekennzeichnet, dass** die Griffelemente (16, 17) als flache Körper ausgebildet sind, die sich bis an die Anschlussstücke (7, 8) des Basiskörpers (1) erstrecken.

13. Vorrichtung nach Anspruch 11 oder 12, **dadurch gekennzeichnet, dass** die Griffelemente (16, 17) an der den Anschlussstücken (7, 8) zugewandten Seiten eine schräg von den Anschlussstücken weg verlaufende Kontur aufweisen.

14. Vorrichtung nach einem der Ansprüche 11 bis 13, **dadurch gekennzeichnet, dass** mindestens eines der Griffelelemente (16, 17) eine erhabene Struktur (19) oder eine muldenförmige Vertiefung oder eine Aussparung aufweist.

15. Vorrichtung nach einem der Ansprüche 1 bis 14, **dadurch gekennzeichnet, dass** mindestens eines der Anschlussstücke (7, 8) des Basiskörpers (1) mit einer erhabenen Struktur (18), insbesondere einem erhabenen Streifen, versehen ist.

16. Vorrichtung nach einem der Ansprüche 1 bis 15, **dadurch gekennzeichnet, dass** ein Verschlussteil (23) zum Verschließen des Einlasskanals (11) des Verschlusskörpers (2) vorgesehen ist.

17. Vorrichtung nach Anspruch 16, **dadurch gekennzeichnet, dass** der Verschlussteil (23) einen Verschlussstopfen (25) aufweist, der unverlierbar an dem Verschlusskörper (2) befestigt ist.

## Claims

1. Apparatus for supplying medical liquids, in particular for applying a medicament to the hose line of a transfer unit for enteral or parenteral nutrition, having
a base body (1), which has an inlet channel (7A) and an outlet channel (8A), and a closure body (2), which is arranged in a rotatable manner in the base body and has an inlet channel (11), wherein the closure body has a first through-passage (10, 10'), which, in a first position, connects the inlet channel of the base body to the outlet channel of the base body, and has a second through-passage (13), which, in a second position, connects the inlet channel of the closure body to the outlet channel of the base body,
**characterized in that**
the closure body (2) is arranged in a rotatable manner in the base body (1) such that, as it rotates, the closure body simultaneously executes an axial lifting movement as a result of said rotation, wherein the first and second through-passages (10, 13) are arranged in the closure body such that, in the first position, the first through-passage (10) connects the inlet channel (7A) of the base body (1) to the outlet channel (8B) of the base body and, in the second position of the closure body, said first through-passage connects the inlet channel (11) of the closure body (2) to the outlet channel (8B) of the base body.

2. Apparatus according to Claim 1, **characterized in that** the first through-passage is a bore (10) in the closure body (2), the longitudinal axis of said bore running transversely to the axis of rotation of the closure body.

3. Apparatus according to Claim 1 or 2, **characterized in that** the second through-passage (13) has a portion (13A) which runs in the direction of the axis of rotation of the closure body and is adjoined by a portion (13B) running transversely to the axis of rotation.

4. Apparatus according to one of Claims 1 to 3, **characterized in that** the outlet of the first through-passage (10) and the outlet of the second through-passage (13) enclose a circumferential angle of 90°.

5. Apparatus according to one of Claims 1 to 4, **characterized in that** the outlet of the first through-passage (10) and the outlet of the second through-passage (13) are arranged at different heights in the axial direction, wherein the distance between the outlet of the first through-passage and the outlet of the second through-passage corresponds to the height of the lifting movement executed by the closure body as it rotates between the first and second positions.

6. Apparatus according to one of Claims 1 to 5, **characterized in that** the base body (1) has an essentially cylindrical component (3), which is adjoined by radially outwardly projecting connection pieces (7, 8), in which the inlet channel and the outlet channel of the base body are formed, and **in that** the closure body (2) has a connection piece (12), which extends transversely to the connection pieces of the base body, wherein the inlet channel (11) of the closure body is formed in the connection piece (12).

7. Apparatus according to Claim 6, **characterized in that** the connection piece (12) of the closure body (2) has a first portion (12A), which is adjoined by a second portion (12B), wherein the inlet channel (11) has a larger internal diameter in the first portion than in the second portion of the connection piece.

8. Apparatus according to Claim 7, **characterized in that** at least one of the two portions (12A, 12B) of the connection piece (12) is of conical design.

9. Apparatus according to one of Claims 1 to 8, **characterized in that** the closure body (2) has an outer portion (28), which engages over the upper portion of the base body (1).

10. Apparatus according to Claim 9, **characterized in that** the portion (28) of the closure body (2), said portion engaging over the upper portion of the base body (1), has at least one groove (14), in which a shoulder (15) of the base body (1) engages, wherein the progression of the groove is such that the closure body executes a lifting movement as it rotates.

11. Apparatus according to one of Claims 1 to 10, **characterized in that** the closure body (2) has two handle elements (16, 17) which project radially from the closure body.

12. Apparatus according to Claim 11, **characterized in that** the handle elements (16, 17) are designed in the form of flat bodies which extend as far as the connection pieces (7, 8) of the base body (1).

13. Apparatus according to Claim 11 or 12, **characterized in that**, on the sides which are directed towards the connection pieces (7, 8), the handle elements (16, 17) have a contour which runs obliquely away from the connection pieces.

14. Apparatus according to one of Claims 11 to 13, **characterized in that** at least one of the handle elements (16, 17) has a raised structure (19) or a hollow-like depression or an aperture.

15. Apparatus according to one of Claims 1 to 14, **characterized in that** at least one of the connection pieces (7, 8) of the base body (1) is provided with a raised structure (18), in particular a raised strip.

16. Apparatus according to one of Claims 1 to 15, **characterized in that** a closure part (23) is provided for closing the inlet channel (11) of the closure body (2).

17. Apparatus according to Claim 16, **characterized in that** the closure part (23) has a closure plug (25), which is fastened in captive fashion on the closure body (2).

## Revendications

1. Dispositif d'amenée de liquides médicaux, en particulier pour la délivrance d'un médicament dans la conduite flexible d'une pompe de nutrition pour la nutrition entérale et parentérale, avec
un corps de base (1), qui présente un canal d'entrée (7A) et un canal de sortie (8A) et un corps de fermeture (2) disposé de façon rotative dans le corps de base, qui présente un canal d'entrée (11),
dans lequel le corps de fermeture présente un premier passage (10, 10'), qui dans une première position relie le canal d'entrée du corps de base au canal de sortie du corps de base, et présente un deuxième passage (13), qui dans une deuxième position relie le canal d'entrée du corps de fermeture au canal de sortie du corps de base,
**caractérisé en ce que**
le corps de fermeture (2) est disposé de façon rotative dans le corps de base (1), de telle manière que le corps de fermeture exécute simultanément lors de la rotation un mouvement de levée axiale dû à cette rotation,
dans lequel le premier et le deuxième passages (10, 13) sont disposés dans le corps de fermeture de telle manière que dans la première position le premier passage (10) relie le canal d'entrée (7A) du corps de base (1) au canal de sortie (8B) du corps de base et dans la deuxième position du corps de fermeture relie le canal d'entrée (11) du corps de fermeture (2) au canal de sortie (8B) du corps de base.

2. Dispositif selon la revendication 1, **caractérisé en ce que** le premier passage est un alésage (10) dans le corps de fermeture (2), dont l'axe longitudinal s'étend transversalement à l'axe de rotation du corps de fermeture.

3. Dispositif selon la revendication 1 ou 2, **caractérisé en ce que** le deuxième passage (13) présente une partie (13A) s'étendant dans la direction de l'axe de rotation du corps de fermeture, à laquelle se raccorde une partie (13B) s'étendant transversalement à l'axe de rotation.

4. Dispositif selon l'une quelconque des revendications 1 à 3, **caractérisé en ce que** la sortie du premier passage (10) et la sortie du deuxième passage (13) forment un angle circonférentiel de 90°.

5. Dispositif selon l'une quelconque des revendications 1 à 4, **caractérisé en ce que** la sortie du premier passage (10) et la sortie du deuxième passage (13) sont disposées à des hauteurs différentes en direction axiale, dans lequel la distance entre la sortie du premier passage et la sortie du deuxième passage correspond à la hauteur du mouvement de levée, que le corps de fermeture exécute lors de la rotation entre la première et la deuxième positions.

6. Dispositif selon l'une quelconque des revendications 1 à 5, **caractérisé en ce que** le corps de base (1) présente une pièce partielle essentiellement cylindrique (3), à laquelle se raccordent des pièces de raccordement (7, 8) saillantes radialement vers l'extérieur, dans lesquelles le canal d'entrée et le canal de sortie du corps de base sont formés, et **en ce que** le corps de fermeture (2) présente une pièce de raccordement (12), qui s'étend transversalement aux pièces de raccordement du corps de base, dans lequel le canal d'entrée (11) du corps de fermeture est formé dans la pièce de raccordement (12).

7. Dispositif selon la revendication 6, **caractérisé en ce que** la pièce de raccordement (12) du corps de fermeture (2) présente une première partie (12A), à laquelle se raccorde une deuxième partie (12B), dans lequel le canal d'entrée (11) dans la première partie a un diamètre intérieur plus grand que dans la deuxième partie de la pièce de raccordement.

8. Dispositif selon la revendication 7, **caractérisé en ce qu'**au moins une des deux parties (12A, 12B) de la pièce de raccordement (12) est de forme conique.

9. Dispositif selon l'une quelconque des revendications 1 à 8, **caractérisé en ce que** le corps de fermeture (2) présente une partie extérieure (28), qui recouvre la partie supérieure du corps de base (1).

10. Dispositif selon la revendication 9, **caractérisé en ce que** la partie (28) du corps de fermeture (2) qui recouvre la partie supérieure du corps de base (1) présente au moins une rainure (14), dans laquelle s'engage un ergot (15) du corps de base (1), dans lequel le tracé de la rainure est réalisé de telle manière que le corps de fermeture exécute un mouvement de levée lors de la rotation.

11. Dispositif selon l'une quelconque des revendications 1 à 10, **caractérisé en ce que** le corps de fermeture (2) présente deux éléments de saisie (16, 17), qui sont saillants radialement à partir du corps de fermeture.

12. Dispositif selon la revendication 11, **caractérisé en ce que** les éléments de saisie (16, 17) sont réalisés sous forme de corps plats, qui s'étendent jusqu'aux pièces de raccordement (7, 8) du corps de base (1).

13. Dispositif selon la revendication 11 ou 12, **caractérisé en ce que** les éléments de saisie (16, 17) présentent sur les côtés tournés vers les pièces de raccordement (7, 8) un contour s'écartant en oblique des pièces de raccordement.

14. Dispositif selon l'une quelconque des revendications 11 à 13, **caractérisé en ce qu'**au moins un des éléments de saisie (16, 17) présente une structure en relief (19) ou un creux en forme de cuvette ou une découpe.

15. Dispositif selon l'une quelconque des revendications 1 à 14, **caractérisé en ce qu'**au moins une des pièces de raccordement (7, 8) du corps de base (1) est dotée d'une structure en relief (8), en particulier d'une bande en relief.

16. Dispositif selon l'une quelconque des revendications 1 à 15, **caractérisé en ce qu'**il est prévu une partie de fermeture (23) pour la fermeture du canal d'entrée (11) du corps de fermeture (2).

17. Dispositif selon la revendication 16, **caractérisé en ce que** la partie de fermeture (23) présente un bouchon de fermeture (25), qui est fixé de façon imperdable au corps de fermeture (2).
